# EUROPEAN PATENT SPECIFICATION

(11) **EP 4 094 700 B1**
(45) Date of publication and mention of the grant of the patent: **14.05.2025**
(21) Application number: 22173495.7
(22) Date of filing: 16.05.2022
(51) Int. Cl.: A61B 17/34, A61F 9/007

(54) **EYEBALL ATTACHMENT TUBE**
AUGAPFELAUFSATZROHR
TUBE DE FIXATION DE GLOBE OCULAIRE

(30) Priority: 28.05.2021 JP 2021089894
(43) Date of publication of application: 30.11.2022
(73) Proprietor: Trabector Co., Inc., Nagoya-shi, Aichi 456-0032 (JP)
(72) Inventor: ICHIKAWA, Kazuo, Nagoya-shi, 4560032 (JP); TODA, Hiroto, Nagoya-shi, 4560032 (JP)
(74) Representative: Ter Meer Steinmeister & Partner

(56) References cited:
- EP-A1- 2 392 274
- EP-A1- 3 701 890
- WO-A1-2013/047473
- US-A1- 2004 073 231
- US-A1- 2008 097 346
- US-A1- 2012 053 505
- US-A1- 2016 213 511
- US-A1- 2019 053 825
- US-B1- 7 846 134

## Description

### BACKGROUND OF THE INVENTION

### Field of the Invention

This disclosure relates to an eyeball attachment tube that is temporarily attached to an outer membrane of an eyeball in ophthalmic surgery for communication between the inside and the outside of the eyeball.

### Description of Related Art

To date, an eyeball attachment tube (also referred to as trocar, cannula) that is temporarily attached to an outer membrane of an eyeball in ophthalmic surgery for communication between the inside and the outside of the eyeball has been known (for example, see Japanese Laid-Open Patent Publication No. 2019-25023, Japanese Patent No. 6697206). This type of eyeball attachment tube has a large diameter portion in which an outer diameter is increased on the proximal end side. When the eyeball attachment tube is attached to an eyeball, the large diameter portion comes into contact with the external surface of the eyeball to act as a stopper, thereby preventing the entirety of the eyeball attachment tube from being inserted into the eyeball.

As described in Japanese Patent No. 6697206, in a case where the eyeball attachment tube is attached for communication with an anterior chamber, the eyeball attachment tube needs to be attached at a border (also referred to as corneosclera or corneal limbus) between a cornea and a sclera, or at a peripheral portion of the cornea. This is because, if the eyeball attachment tube is attached near the center of a cornea, the cornea may be deformed. In a case where the eyeball attachment tube is attached at the border or the peripheral portion, if the eyeball attachment tube punctures the external surface of the border or the peripheral portion so as to be perpendicular thereto, an iris or a crystalline lens may be injured. In order to prevent this, when the anteroposterior direction is defined such that the corneal apex side is the anterior side and the retina center side is the posterior side, the eyeball attachment tube needs to be attached so as to be oriented in the horizontal direction crossing the anteroposterior direction (more specifically, slightly oriented diagonally forward). The peripheral portion of the cornea represents a portion of the cornea which is slightly closer to the cornea center side than the border is. The "peripheral portion of a cornea" may be defined as including the border or the "border between a cornea and a sclera" may be defined as including the peripheral portion.

However, in a case where the eyeball attachment tube is attached so as to deviate relative to the direction perpendicular to the external surface of the eyeball, for example, in a case where the eyeball attachment tube is attached in the horizontal direction at the border or the peripheral portion, a gap may be generated between the large diameter portion of the eyeball attachment tube and the external surface of the eyeball, and the eyeball attachment tube becomes unstable and may be removed from the eyeball.

WO 2013/047473 discloses an eyeball attachment tube that is temporarily attached to an outer membrane of an eyeball.

This disclosure has been made in view of the aforementioned problem, and an object of this disclosure is to inhibit an eyeball attachment tube from becoming unstable in a case where the eyeball attachment tube is attached so as to deviate relative to the direction perpendicular to an external surface of an eyeball.

### SUMMARY OF THE INVENTION

The invention is defined by claim 1. Further embodiments of the invention are defined by the dependent claims. An eyeball attachment tube of this disclosure is directed to an eyeball attachment tube that is temporarily attached to an outer membrane of an eyeball in ophthalmic surgery for communication between an inside and an outside of the eyeball.

The eyeball attachment tube includes:
a tubular portion formed in a tubular shape, the tubular portion configured to be in a state of puncturing the outer membrane when the eyeball attachment tube is attached to the outer membrane; and
a large diameter portion disposed on a proximal end side of the tubular portion, the large diameter portion having an outer diameter larger than an outer diameter of the tubular portion, the large diameter portion configured to be exposed externally from the outer membrane when the eyeball attachment tube is attached to the outer membrane.

An end portion, in a parallel direction to a center axis of the tubular portion, of the large diameter portion on the tubular portion side is shaped so as to have a height difference in the parallel direction between one end and another end in a direction perpendicular to the center axis in a side view.

An entirety of the end portion is formed as an inclined plane relative to the center axis.

An end portion, of the large diameter portion, on an opposite side to the end portion on the tubular portion side in the parallel direction is formed as a plane perpendicular to the center axis.

In this configuration, in a side view, the end portion of the large diameter portion is shaped so as to have a height difference, in the direction parallel to the center axis, between one end and the other end in the direction perpendicular to the center axis of the tubular portion. Therefore, in a case where the eyeball attachment tube is attached in a direction deviating relative to the direction perpendicular to the external surface of the eyeball, a gap between the external surface of the eyeball and the large diameter portion can be reduced by the height difference. Thus, the eyeball attachment tube can be inhibited from becoming unstable. The entirety of the end portion of the large diameter portion on the tubular portion side is formed as the plane inclined relative to the center axis. Therefore, as compared with a case where a part of the end portion of the large diameter portion is formed as an inclined plane, the eyeball attachment tube can be further inhibited from becoming unstable when attached to the external surface of the eyeball. Specifically, the large diameter portion can be inhibited from rolling on an eyeball, and the eyeball attachment tube attached to the eyeball can be inhibited from becoming unstable in the clockwise or counterclockwise direction around a connection portion between the large diameter portion and the tubular portion in a side view. Furthermore, the end portion of the large diameter portion on the side opposite to the inclined surface side is formed as the plane perpendicular to the center axis. Therefore, the eyeball attachment tube can be attached to the external surface of an eyeball by using a conventional instrument described in Patent Documents (for example, Japanese Laid-Open Patent Publication No. 2019-25023, Japanese Patent No. 6697206). In other words, an instrument for attachment can be shared between an instrument for attaching the eyeball attachment tube of this disclosure to an eyeball and an instrument for attaching a conventional eyeball attachment tube to an eyeball.

### BRIEF DESCRIPTION OF THE DRAWINGS

FIG. 1 is a side view of an eyeball attachment tube;
FIG. 2 illustrates the eyeball attachment tube as viewed in a direction A in FIG. 1 in a plane orthogonal to the center axis of the eyeball attachment tube;
FIG. 3 illustrates the eyeball attachment tube as viewed from the upper side B in the sheet surface shown in FIG. 1;
FIG. 4 illustrates the eyeball attachment tube as viewed from the lower side C in the sheet surface shown in FIG. 1;
FIG. 5 is a perspective view of the eyeball attachment tube;
FIG. 6 is a first cross-sectional view of the eyeball attachment tube as viewed in the same direction as in FIG. 1 and taken along a line VI-VI in FIG. 2;
FIG. 7 is a second cross-sectional view of the eyeball attachment tube as taken at the plane (plane indicated by a line VII-VII in FIG. 2) perpendicular to the cross-section in FIG. 6;
FIG. 8 illustrates a state where the eyeball attachment tube is being attached to a corneosclera of an anterior segment;
FIG. 9 illustrates a state where the eyeball attachment tube is attached to a corneosclera of an anterior segment;
FIG. 10 is a side view of an eyeball attachment tube according to a first modification;
FIG. 11 is a first cross-sectional view of an eyeball attachment tube according to a second modification, in a side view;
FIG. 12 is a second cross-sectional view of the eyeball attachment tube according to the second modification, illustrating a cross-section perpendicular to the cross-section in FIG. 11;
FIG. 13 illustrates a state where an eyeball attachment tube is attached to a sclera; and
FIG. 14 is a side view of a conventional eyeball attachment tube.

### DESCRIPTION OF THE PREFERRED EMBODIMENTS

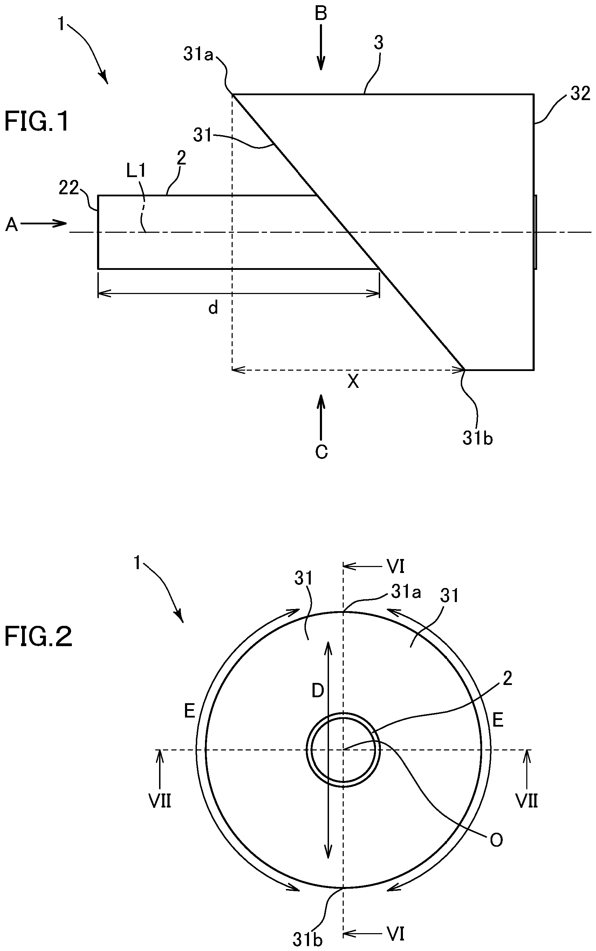

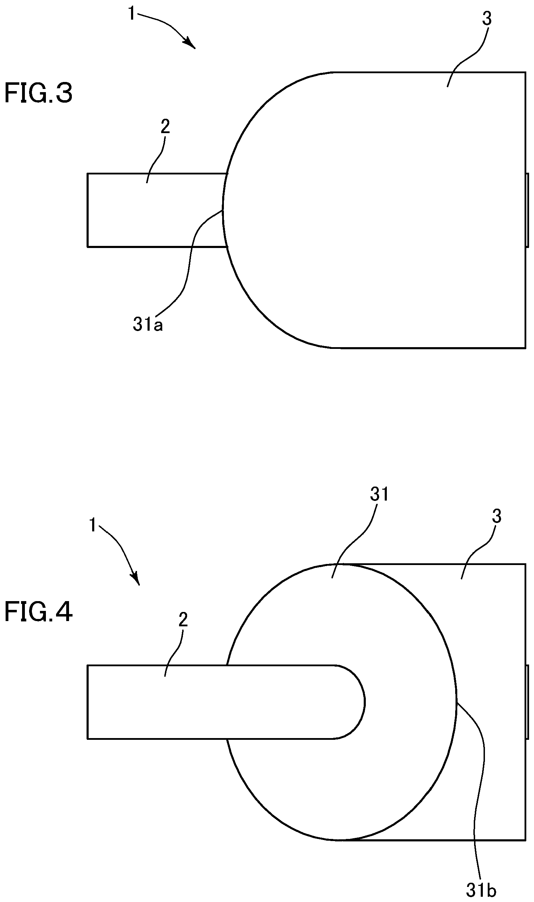

An embodiment of this disclosure will be described below with reference to the drawings. An eyeball attachment tube 1 (cannula, trocar) (hereinafter, may be simply referred to as attachment tube) shown in FIG. 1 to FIG. 7 is temporarily attached to a corneosclera of an eyeball in ophthalmic surgery such as cataract surgery and glaucoma surgery, for communication between an anterior chamber and the outside of the eyeball. The attachment tube 1 includes a tubular portion 2 formed in a linear tubular shape, and a large diameter portion 3 that is disposed on the proximal end side of the tubular portion 2 and that has an outer diameter larger than an outer diameter of the tubular portion 2.

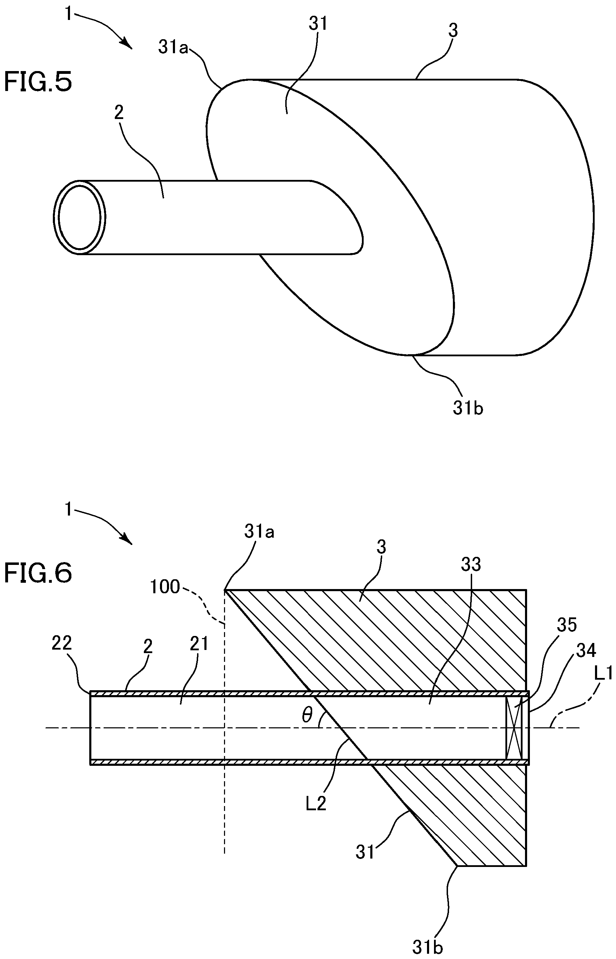

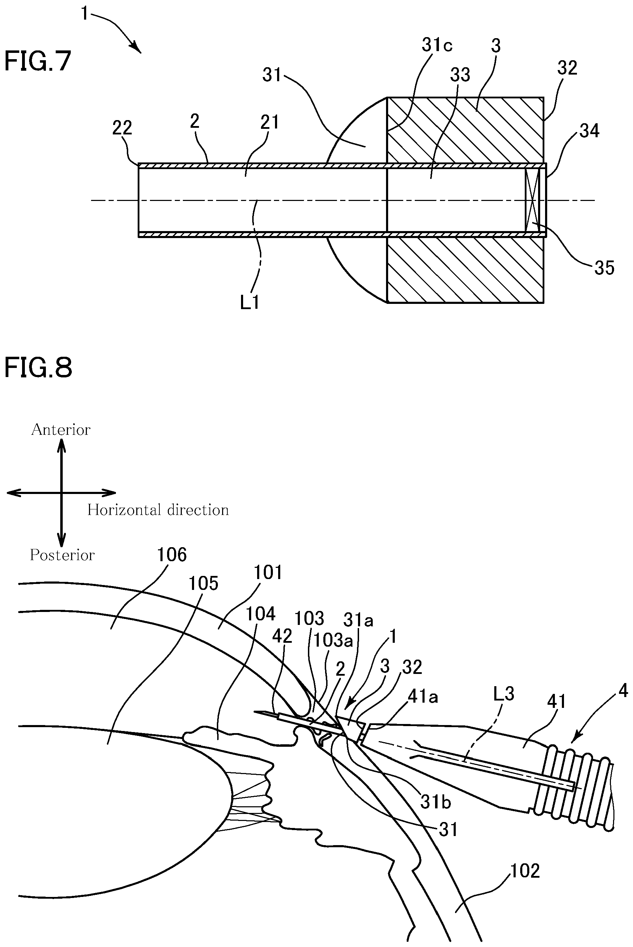

A center axis L1 of the tubular portion 2 linearly extends. The tubular portion 2 is formed in such a shape (that is, cylindrical shape) as to have a circular outer circumferential line and a circular inner circumferential line as viewed in a plane (direction in FIG. 2) to which the center axis L1 is orthogonal. However, the tubular portion 2 may be formed in a tubular shape that has an outer circumferential line and an inner circumferential line having other shapes. The tubular portion 2 has a through hole 21 formed therein so as to penetrate from one end to the other end in the direction in which the center axis L1 extends (see FIG. 6, FIG. 7). A length d (see FIG. 1) of a portion of the tubular portion 2 which is exposed from the large diameter portion 3 is set to be greater than the thickness of a corneosclera and set such that a leading end 22 of the tubular portion 2 does not protrude from an anterior chamber in a state where the corneosclera is punctured by the tubular portion 2. Specifically, the length d is set to be, for example, larger than or equal to 2 mm and less than or equal to 10 mm. From the viewpoint of preventing deformation of an eyeball when a corneosclera is punctured by the tubular portion 2 or the viewpoint of naturally closing a hole formed in the corneosclera when the tubular portion 2 is removed from the corneosclera, the outer diameter of the tubular portion 2 is preferably small, and can be, for example, less than or equal to 1 mm.

The large diameter portion 3 is formed in a tubular shape having an outer diameter larger than the outer diameter of the tubular portion 2. In this embodiment, the large diameter portion 3 is formed in such a shape (that is, cylindrical shape) as to have a circular outer circumferential line that is concentric with the outer circumferential line of the tubular portion 2 as viewed in a plane (direction in FIG. 2) to which the center axis L1 of the tubular portion 2 is orthogonal. However, the large diameter portion 3 may be formed in a tubular shape having another shape. The large diameter portion 3 is connected to the proximal end side portion of the tubular portion 2 in a state where, for example, a part of the proximal end side portion of the tubular portion 2 is fitted into the large diameter portion 3. The center axis of the large diameter portion 3 is coaxial with the center axis L1 of the tubular portion 2. Inside the large diameter portion 3, a channel 33 communicating with the channel 21 inside a portion of the tubular portion 2 which is exposed from the large diameter portion 3 is formed (see FIG. 6). The channel 33 is formed to be coaxial with the channel 21 of the tubular portion 2. The channel 33 may be formed by a channel in a portion of the tubular portion 2 which is fitted into the large diameter portion 3 or formed of a material other than a material of the tubular portion 2.

The large diameter portion 3 is formed in, for example, a shape obtained by diagonally cutting one end side portion, in the axial direction, of a cylindrical member. Specifically, the entirety of an end portion 31 of the large diameter portion 3 on the leading end 22 side (the portion of the tubular portion 2 which is exposed from the large diameter portion 3) of the tubular portion 2 in the axis L1 direction is formed as a plane diagonal relative to the center axis L1. In other words, the entirety of the end portion 31 is angled relative to an imaginary plane 100 (see FIG. 6) perpendicular to the center axis L1. In still other words, the end portion 31 is formed in a shape having, in the direction parallel to the center axis L1, a height difference x between one end 31b side and the other end 31a side in the direction perpendicular to the center axis L1 in the side view in FIG. 1. Specifically, the end portion 31 is shaped so as to be gradually displaced to (approach) the leading end 22 of the tubular portion 2 from the one end 31b side toward the other end 31a side.

More specifically, in the side view in FIG. 1, the end portion 31 is formed so as to render an outline (straight line) diagonal relative to the center axis L1. The end portion 31 has a portion 31a (referred to as portion closest to the leading end) closest to the leading end 22 and a portion 31b (referred to as portion closest to the proximal end) closest to the proximal end 32 of the large diameter portion 3 (see FIG. 1 to FIG. 6). The portions 31a, 31b are disposed on 180° opposite sides in a circle having, as the center of the circle, the center O (point on the center axis L1) of the tubular portion 2 as viewed in the direction in FIG. 2. The end portion 31 is formed so as to be gradually displaced toward the proximal end 32 as the end portion 31 approaches the portion 31b closest to the proximal end from the portion 31a closest to the leading end along the radial direction D (see FIG. 2) in the circle having, as the center of the circle, the center O of the tubular portion 2. The end portion 31 is formed so as to be gradually displaced toward the proximal end 32 as the end portion 31 approaches the portion 31b closest to proximal end from the portion 31a closest to the leading end along the circumferential direction E (see FIG. 2) in the circle.

The cross-section in FIG. 6 represents a cross-section obtained by cutting the attachment tube 1 at a plane (plane represented by a line VI-VI in FIG. 2) including therein the center axis L1, the portion 31a closest to the leading end, and the portion 31b closest to the proximal end. FIG. 7 illustrates a cross-section obtained by cutting the attachment tube 1 at a plane (plane represented by a line VII-VII in FIG. 2) perpendicular to the cross-section in FIG. 6. In FIG. 7, a cross-sectional line 31c of the end portion 31 is perpendicular to the center axis L1. A cross-sectional line of the end portion 31 formed by cutting the attachment tube 1 at any plane including the center axis L1 therein is rendered as a straight line diagonal relative to the center axis L1 except for the cross-sectional line 31c in FIG. 7.

As shown in FIG. 6, an angle θ forming an acute angle in angles between the center axis L1 and a straight line L2 passing through the portion 31a closest to the leading end and the portion 31b closest to the proximal end is defined as an inclination angle of the end portion 31 relative to the center axis L1. The inclination angle θ is greater than 0° and less than 90°. Specifically, the inclination angle θ is set as an angle corresponding to inclination of the external surface of the corneosclera, and is set to be, for example, greater than or equal to 20° and less than or equal to 70°. More specifically, when the anteroposterior direction is defined such that the corneal apex side in an anterior segment is the anterior side, and the retina center side is the posterior side, and the direction perpendicular to the anteroposterior direction is defined as the horizontal direction, the inclination angle of the corneosclera relative to the horizontal direction is, for example, 40 to 50°. In a case where the attachment tube 1 is attached in the horizontal direction, the inclination angle θ is set as an angle (40 to 50°) that is approximately equal to the inclination angle of the corneosclera. For example, in a case where the attachment tube 1 is attached so as to be oriented forward by about 5° relative to the horizontal direction, the inclination angle θ is set as an angle (35 to 45°) obtained by subtracting the forward inclining angle (5°) from the inclination angle (40 to 50°) of the corneosclera. The end portion 31 functions as a portion that is in contact with the external surface of the outer membrane when the attachment tube 1 is attached to the outer membrane of the eyeball.

The proximal end 32, of the large diameter portion 3, which is an end portion on the side opposite to the end portion 31 side in the direction parallel to the axis L1 is formed as a plane perpendicular to the center axis L1. In the proximal end 32, an opening 34 for the channel 33 of the large diameter portion 3 is formed.

The large diameter portion 3 has a valve 35 for inhibiting liquid from flowing out from the opening 34 through the channels 21, 33. The valve 35 is disposed at a position of the opening 34 of the channel 33 or disposed in front of the opening 34. For example, a member having a slit formed in an elastic material such as rubber is disposed as the valve 35 so as to close the channel 33 in the large diameter portion 3. The slit is elastically deformed when a surgical instrument or the like is inserted into the attachment tube 1, whereby the valve 35 opens. The slit is restored to an original state when the instrument is removed from the attachment tube 1, whereby the valve 35 is closed.

The attachment tube 1 is attached to a border 103 (corneosclera) between a cornea 101 and a sclera 102 of an eyeball of a subject to be operated in ophthalmic surgery, as shown in FIG. 8. As an instrument 4 for attaching the attachment tube 1 to the corneosclera 103, the instrument similar to that disclosed in Japanese Laid-Open Patent Publication No. 2019-25023 or Japanese Patent No. 6697206 is used. The instrument 4 includes a bar-like handle portion 41, and a needle portion 42 connected to a leading end 41a of the handle portion 41. The leading end 41a is formed as a plane perpendicular to an axis L3 of the handle portion 41 and the needle portion 42.

In a procedure for attaching the attachment tube 1 to the corneosclera 103, firstly, the needle portion 42 is caused to pass through the hole 21 in the attachment tube 1 to attach the attachment tube 1 to the instrument 4. At this time, the proximal end 32 of the large diameter portion 3 of the attachment tube 1 is brought into contact with the leading end 41a of the handle portion 41, whereby a part of the needle portion 42 from the leading end protrudes from the attachment tube 1 while the attachment tube 1 is held at the leading end 41a and the needle portion 42. In FIG. 8, for facilitating understanding of the structure of the instrument 4, a gap between the leading end 41a of the handle portion 41 and the proximal end 32 of the large diameter portion 3 is shown. However, the leading end 41a and the proximal end 32 are actually in contact with each other.

Thereafter, a person (doctor) who performs the surgery holds the handle portion 41 and punctures the corneosclera 103 with the needle portion 42. At this time, the needle portion 42 is oriented almost in the horizontal direction (specifically, oriented slightly forward (toward cornea apex side) relative to the horizontal direction) in order to prevent the needle portion 42 from injuring an iris 104 or a crystalline lens 105.

The person who performs the surgery inserts the tubular portion 2 of the attachment tube 1 into the corneosclera 103 while puncturing the corneosclera 103 with the needle portion 42 to form a hole in the corneosclera 103. At this time, while the tubular portion 2 is positioned forward of the iris 104, the tubular portion 2 is inserted until the large diameter portion 3 comes into contact with an external surface 103a of the corneosclera 103. The portion 31a, closest to the leading end, of the large diameter portion 3 is brought into contact with the external surface 103a on the cornea 101 side (anterior side), and the portion 31b closest to the proximal end is brought into contact with the external surface 103a on the sclera 102 side (posterior side). The person who performs the surgery may adjust the orientation of the attachment tube 1 around the axis L1 by rotating the instrument 4 around the axis of the needle portion 42.

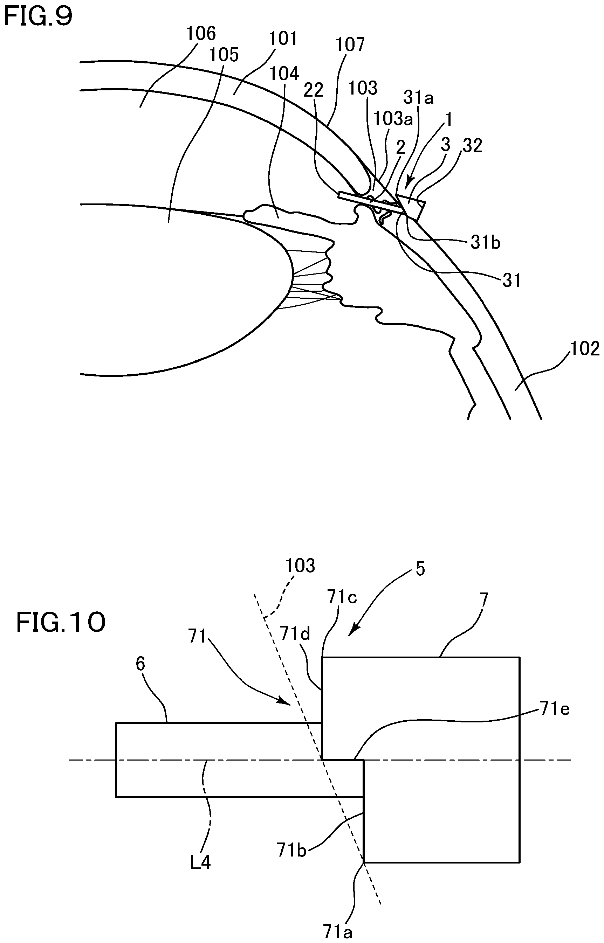

Thereafter, the instrument 4 (the needle portion 42) is retracted (pulled) from the corneosclera 103, whereby only the attachment tube 1 is indwelt in the corneosclera 103 (see FIG. 9). When the instrument 4 is retracted, the large diameter portion 3 of the attachment tube 1 may be held by tweezers or the like in order to prevent removal of the attachment tube 1 from the corneosclera 103.

As shown in FIG. 9, in a state where the attachment tube 1 is attached to the corneosclera 103, the tubular portion 2 is in a state of puncturing the corneosclera 103, and the large diameter portion 3 is in a state of being exposed externally from the corneosclera 103. Furthermore, the end portion 31 of the large diameter portion 3 is in contact with the external surface 103a of the corneosclera 103. Moreover, the leading end 22 of the tubular portion 2 is positioned in an anterior chamber interior 106 (see FIG. 9). The anterior chamber interior 106 is a region between the cornea 101 and the crystalline lens 105, and is a region forward of the iris 104.

During the succeeding surgery, a surgical instrument (for example, instrument for resecting a tissue in the eye) is inserted through the attachment tube 1 into the anterior chamber interior 106, or liquid (water) is injected through the attachment tube 1 into the anterior chamber interior 106 such that a pressure (intraocular pressure) of the anterior chamber interior 106 is maintained constant. As glaucoma surgery, for example, trabeculectomy is performed so as to improve flow of aqueous humor and lower an abnormally high intraocular pressure. In this case, for example, an instrument (cutting tool) for the trabeculectomy is inserted through the attachment tube 1 into the anterior chamber interior 106.

When the surgery ends, the large diameter portion 3 of the attachment tube 1 is held by tweezers or the like and pulled, whereby the attachment tube 1 is removed from the eyeball. Thereafter, suture is performed for closing the hole formed in the corneosclera 103 as necessary.

Thus, in this embodiment, the end portion 31 of the large diameter portion 3 on the tubular portion 2 side is formed as a plane inclined relative to the center axis L1 of the tubular portion 2. Therefore, in a case where the attachment tube 1 is attached to the corneosclera 103 almost in the horizontal direction, the entirety of the end portion 31 can be brought into contact with the external surface 103a of the corneosclera 103. Thus, for example, when a surgical instrument having been inserted in the anterior chamber interior 106 through the attachment tube 1 is moved, the attachment tube 1 can be inhibited from becoming unstable, and the attachment tube 1 can be inhibited from being removed from the corneosclera 103.

The proximal end 32 of the large diameter portion 3 is formed as a plane perpendicular to the center axis L1. Therefore, when the attachment tube 1 is attached by using the instrument 4 (see FIG. 8), an area in which the leading end 41a of the handle portion 41 and the proximal end 32 of the large diameter portion 3 are in contact with each other can be increased, and attachment of the attachment tube 1 with use of the instrument 4 is facilitated.

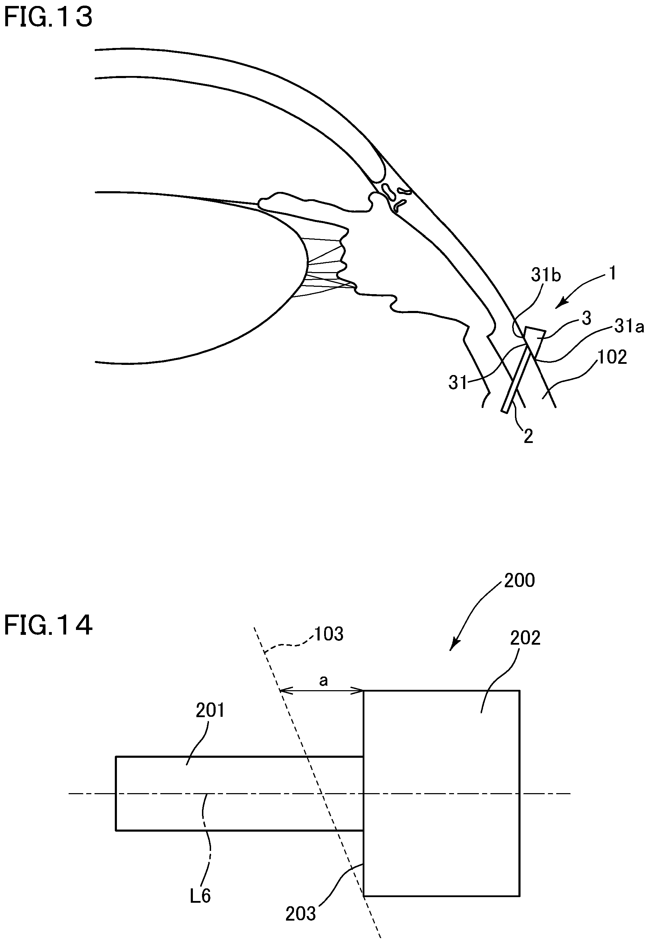

Meanwhile, in a conventional attachment tube 200 shown in FIG. 14, an end portion 203 of a large diameter portion 202 is formed so as to be perpendicular to a center axis L6 of a tubular portion 201. Therefore, when the attachment tube 200 is attached to the corneosclera 103, a gap a is formed between the external surface of the corneosclera 103 and the large diameter portion 202, and the attachment tube 200 becomes unstable and is likely to be removed from the corneosclera 103. In FIG. 14, the outline of the external surface of the corneosclera 103 is indicated by dotted lines.

This disclosure is not limited to the above-described embodiment, and various modifications can be made. In this embodiment, an example in which the end portion of the large diameter portion is an inclined plane is described. However, the end portion of the large diameter portion may not necessarily be formed as an inclined plane when a height difference is formed, in the direction parallel to the center axis, between one of the end portions of the large diameter portion and the other thereof in the direction perpendicular to the center axis in a side view. FIG. 10 shows an example in which the end portion of the large diameter portion is formed in a shape other than the inclined plane. An end portion 71 of a large diameter portion 7 of an attachment tube 5 shown in FIG. 10 is formed in a shape displaced toward the leading end of a tubular portion 6 in a step-like manner (in other words, stepwise) as the end portion 71 approaches the other end 71c from one end 71a in the direction perpendicular to the center axis L4 of the tubular portion 6. Specifically, a portion 71b of the end portion 71 on the one end 71a side in the direction perpendicular to the center axis L4 of the tubular portion 6 is formed as a plane perpendicular to the center axis L4. A portion 71d of the end portion 71 on the other end 71c side in the direction perpendicular to the center axis L4 is formed as a plane perpendicular to the center axis L4. A stepped portion 71e is formed between the portions 71b and 71d so as to be parallel to the center axis L4. For example, a position of the stepped portion 71e may overlap the center axis L4 in the side view in FIG. 10. The stepped portion 71e is set to have a size corresponding to the inclination of the external surface of the corneosclera 103. Specifically, the size of the stepped portion 71e is set such that, when the attachment tube 5 is attached to the corneosclera 103, at least a part of the portion 71d of the end portion 71, which is close to the leading end of the tubular portion 6, is in contact with the external surface of the corneosclera 103 on the cornea side, and at least a part of the portion 71b of the end portion 71, which is close to the proximal end of the large diameter portion 7, is in contact with the external surface of the corneosclera 103 on the sclera side.

Also in such a structure, when the attachment tube 5 is attached to the corneosclera 103, the portion 71d on the leading end side is brought into contact with the corneosclera 103 on the cornea side, and the portion 71b on the proximal end side is brought into contact with the corneosclera 103 on the sclera side, whereby a gap between the large diameter portion 7 and the corneosclera 103 can be reduced as compared with a conventional structure in FIG. 14. In FIG. 10, the outline of the external surface of the corneosclera 103 is indicated by a dotted line.

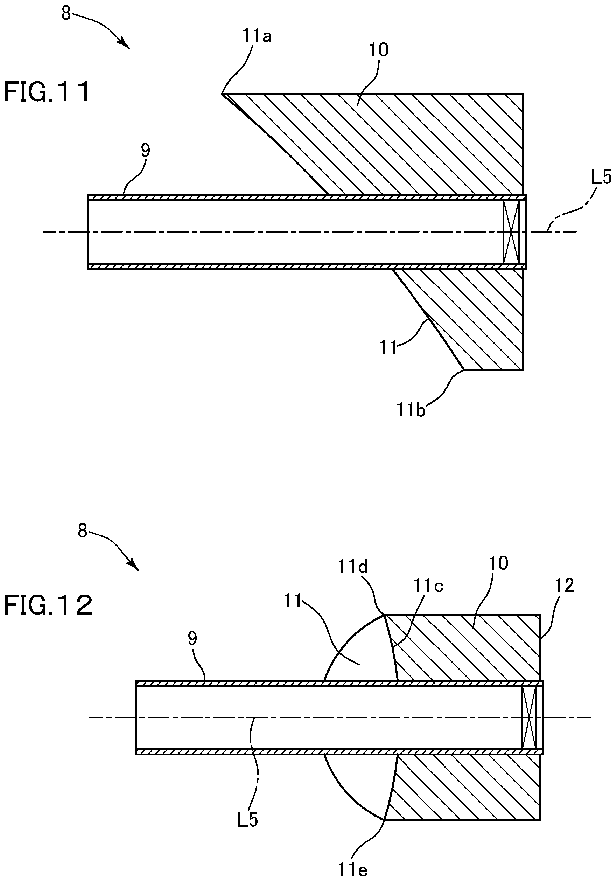

In the above-described embodiment (example shown in FIG. 1 to FIG. 7), an example in which the end portion of the large diameter portion is a plane inclined relative to the center axis, is described. However, as shown in FIG. 11 and FIG. 12, the end portion of the large diameter portion may be formed as a recessed curved surface that is inclined relative to the center axis. An end portion 11 of a large diameter portion 10 of an attachment tube 8 shown in FIG. 11 and FIG. 12 is formed as a recessed curved surface that is inclined relative to a center axis L5 of a tubular portion 9. Specifically, as viewed on the cross-section in FIG. 11, the end portion 11 is gradually displaced toward the leading end of the tubular portion 9 as the end portion 11 approaches the other end 11a from one end 11b in the direction perpendicular to the center axis L5, and is formed so as to render a curved line diagonal relative to the center axis L5. FIG. 11 shows a first cross-section obtained by cutting the attachment tube 8 at the same position as the position of the line VI-VI in FIG. 2.

FIG. 12 shows a second cross-section obtained by cutting the attachment tube 8 at the same position as the position of the line VII-VII in FIG. 2. In other words, FIG. 12 shows a second cross-section obtained by cutting the attachment tube 8 at the plane, perpendicular to the sheet surface in FIG. 11, including the center axis L5 therein. Also on the cross-section shown in FIG. 12, a cross-sectional line 11c rendered by the end portion 11 is a curved line. Specifically, the cross-sectional line 11c is gradually displaced toward a proximal end 12 of the large diameter portion 10 so as to form a curved line shape as the cross-sectional line 11c approaches the center axis L5 from one end portion 11d of the cross-sectional line 11c, and gradually displaced toward the leading end of the tubular portion 9 so as to form a curved line shape as the cross-sectional line 11c approaches the other end portion 11e of the cross-sectional line 11c from the center axis L5. Both of the end portions 11d and 11e of the cross-sectional line 11c are disposed at the same positions (the same axial positions) in the direction in which the center axis L5 extends.

A curvature of the curved surface formed by the end portion 11 is defined so as to be the same as a curvature of an outer membrane (for example, corneosclera) of an eyeball. The center of the curvature of the curved surface formed by the end portion 11 is defined in a region on the tubular portion 9 side outside the end portion 11.

In the attachment tube 8 shown in FIG. 11 and FIG. 12, the end portion 11 of the large diameter portion 10 is formed as a recessed curved surface that is inclined relative to the center axis L5, so that the end portion 11 and an external surface of an eyeball having a curved surface shape can be more advantageously brought into contact with each other.

In the above-described embodiment, an example in which the attachment tube is attached to a corneosclera is described. However, the attachment tube of this disclosure may be attached to a cornea peripheral portion 107 (see FIG. 9) that is slightly closer to the center of the cornea than the corneosclera is. Furthermore, in vitreous surgery, the attachment tube of this disclosure may be attached to a sclera for communication between the vitreous body region and the outside of the eyeball. FIG. 13 illustrates an example in which the same attachment tube 1 as in FIG. 1 is attached to the sclera 102. In FIG. 13, the attachment tube 1 is attached to the sclera 102 such that the tubular portion 2 is oriented toward a retina. In this case, the portion 31a, closest to the leading end, of the end portion 31 of the large diameter portion 3 may be brought into contact with the external surface of the sclera 102 at the posterior side (side farther from the cornea), and the portion 31b closest to the proximal end may be brought into contact with the external surface of the sclera 102 at the anterior side (side closer to the cornea). In this structure, the large diameter portion 3 and the sclera 102 can be advantageously brought into contact with each other, so that the attachment tube 1 can be inhibited from becoming unstable.

### DESCRIPTION OF THE REFERENCE CHARACTERS

1, 5, 8 eyeball attachment tube
2, 6, 9 tubular portion
3, 7, 10 large diameter portion
31, 71, 11 end portion of large diameter portion on tubular portion side
32, 12 proximal end of large diameter portion

The invention is defined by the following claims.

## Claims

1. An eyeball attachment tube (1, 8) that is temporarily attached to an outer membrane of an eyeball in ophthalmic surgery for communication between an inside and an outside of the eyeball, the eyeball attachment tube (1, 8) comprising:
a tubular portion (2, 9) formed in a tubular shape, the tubular portion (2, 9) configured to be in a state of puncturing the outer membrane when the eyeball attachment tube (1, 8) is attached to the outer membrane; and
a large diameter portion (3, 10) disposed on a proximal end side of the tubular portion (2, 9), the large diameter portion (3, 10) having an outer diameter larger than an outer diameter of the tubular portion (2, 9), the large diameter portion (3, 10) configured to be exposed externally from the outer membrane when the eyeball attachment tube (1, 8) is attached to the outer membrane, wherein
an end portion (31, 11), in a parallel direction to a center axis (L1, L5) of the tubular portion (2, 9), of the large diameter portion (3, 10) on the tubular portion (2, 9) side is shaped so as to have a height difference in the parallel direction between one end (31a, 11a) and another end (31b, 11b) in a direction perpendicular to the center axis (L1, L5) in a side view,
an entirety of the end portion (31, 11) is formed as an inclined plane relative to the center axis (L1, L5), and
an end portion (32, 12), of the large diameter portion (3, 10), on an opposite side to the end portion (31, 11) on the tubular portion (2, 9) side in the parallel direction is formed as a plane perpendicular to the center axis (L1, L5), wherein the large diameter portion (3, 10) includes a valve (35) for inhibiting liquid from flowing out from an opening (34) formed in the end portion (32, 12) on the opposite side,
wherein the valve (35) is a member having a slit formed in an elastic material so as to close a channel (33) in the large diameter portion (3, 10).

2. The eyeball attachment tube (1) according to claim 1, wherein the inclined plane (31) is a flat plane.

3. The eyeball attachment tube (8) according to claim 1, wherein
the inclined plane (11) is a recessed curved surface,
on a first cross-section, as viewed in a same direction as in the side view, obtained by cutting the eyeball attachment tube (8) at a plane including the center axis (L5) therein, the recessed curved surface (11) is formed so as to be gradually displaced toward a leading end of the tubular portion (9) as the recessed curves surface (11) approaches another end (11b) side from one end (11a) side in the direction perpendicular to the center axis (L5), and to render a curved line diagonal relative to the center axis (L5), and
on a second cross-section, obtained by cutting the eyeball attachment tube (8) at a plane perpendicular to the first cross-section, including the center axis (L5) therein, a cross-sectional line (11c) rendered by the recessed curved surface (11) is gradually displaced toward the end portion (12) on the opposite side so as to be curved-line-shaped as the cross-sectional line (11c) approaches the center axis (L5) from one of end portions (11d, 11e) of the cross-sectional line (11c), and gradually displaced toward the leading end of the tubular portion (9) so as to be curved-line-shaped as the cross-sectional line (11c) approaches another of the end portions (11d, 11e) of the cross-sectional line (11c) from the center axis (L5) side, and both the end portions (11d, 11e) of the cross-sectional line (11c) are disposed at same positions in a direction in which the center axis (L5) extends.

## Patentansprüche

1. Augapfel-Befestigungsrohr (1, 8), das bei der Augenchirurgie vorübergehend an einer äußeren Membran eines Augapfels angebracht wird, um eine Verbindung zwischen einer Innenseite und einer Außenseite des Augapfels herzustellen, wobei das Augapfel-Befestigungsrohr (1, 8) umfasst:
einen röhrenförmigen Abschnitt (2, 9), der in einer röhrenförmigen Form ausgebildet ist, wobei der röhrenförmige Abschnitt (2, 9) so konfiguriert ist,
dass er sich in einem Zustand befindet, in dem er die äußere Membran durchsticht, wenn das Augapfel-Befestigungsrohr (1, 8) an der äußeren Membran befestigt ist, und
einen Abschnitt (3, 10) mit großem Durchmesser, der an einer proximalen Endseite des röhrenförmigen Abschnitts (2, 9) angeordnet ist, wobei der Abschnitt (3, 10) mit großem Durchmesser einen Außendurchmesser aufweist, der größer ist als ein Außendurchmesser des röhrenförmigen Abschnitts (2, 9), wobei der Abschnitt (3, 10) mit großem Durchmesser so konfiguriert ist, dass er von der äußeren Membran nach außen freiliegt, wenn das Augapfel-Befestigungsrohr (1, 8) an der äußeren Membran befestigt ist,
wobei
ein Endabschnitt (31, 11) des Abschnitts (3, 10) mit großem Durchmesser in einer parallelen Richtung zu einer Mittelachse (L1, L5) des röhrenförmigen Abschnitts (2, 9) auf der Seite des röhrenförmigen Abschnitts (2, 9) so geformt ist, dass er einen Höhenunterschied in der parallelen Richtung zwischen einem Ende (31a, 11a) und einem anderen Ende (31b, 11b) in einer Richtung senkrecht zu der Mittelachse (L1, L5) in einer Seitenansicht aufweist,
der gesamte Endabschnitt (31, 11) als eine schiefe Ebene relativ zur Mittelachse (L1, L5) ausgebildet ist, und
ein Endabschnitt (32, 12) des Abschnitts (3, 10) mit großem Durchmesser auf einer Seite, die dem Endabschnitt (31, 11) auf der Seite des röhrenförmigen Abschnitts (2, 9) in der parallelen Richtung gegenüberliegt, als eine Ebene senkrecht zur Mittelachse (L1, L5) ausgebildet ist,
wobei der Abschnitt (3, 10) mit großem Durchmesser ein Ventil (35) enthält, um zu verhindern, dass Flüssigkeit aus einer Öffnung (34), die in dem Endabschnitt (32, 12) auf der gegenüberliegenden Seite ausgebildet ist, herausfließt,
wobei das Ventil (35) ein Element ist, das einen Schlitz aufweist, der in einem elastischen Material ausgebildet ist, um einen Kanal (33) in dem Abschnitt (3, 10) mit großem Durchmesser zu schließen.

2. Augapfel-Befestigungsrohr (1) nach Anspruch 1, wobei die schiefe Ebene (31) eine flache Ebene ist.

3. Augapfel-Befestigungsrohr (8) nach Anspruch 1, wobei
die schiefe Ebene (11) eine vertiefte gekrümmte Oberfläche ist,
auf einem ersten Querschnitt, in derselben Richtung wie in der Seitenansicht betrachtet, der durch Schneiden des Augapfel-Befestigungsrohrs (8) in einer Ebene erhalten wird, die die Mittelachse (L5) darin enthält, die vertiefte gekrümmte Oberfläche (11) so ausgebildet ist, dass sie allmählich zu einem vorderen Ende des röhrenförmigen Abschnitts (9) hin verschoben ist, wenn sich die vertiefte gekrümmte Oberfläche (11) von einem Ende (11a) in der Richtung senkrecht zur Mittelachse (L5) einer anderen Endseite (11b) nähert, und eine gekrümmte Linie diagonal relativ zur Mittelachse (L5) bildet, und
auf einem zweiten Querschnitt, der durch Schneiden des Augapfel-Befestigungsrohrs (8) in einer Ebene senkrecht zum ersten Querschnitt erhalten wird, die die Mittelachse (L5) darin enthält, eine Querschnittslinie (11c), die durch die vertiefte gekrümmte Oberfläche (11) gebildet wird, allmählich in Richtung des Endabschnitts (12) auf der gegenüberliegenden Seite verschoben wird, so dass sie kurvenförmig ist, wenn sich die Querschnittslinie (11c) der Mittelachse (L5) von einem der Endabschnitte (11d, 11e) der Querschnittslinie (11c) nähert, und allmählich in Richtung des vorderen Endes des röhrenförmigen Abschnitts (9) verschoben wird, so dass sie kurvenförmig ist, wenn sich die Querschnittslinie (11c) einem anderen der Endabschnitte (11d, 11e) der Querschnittslinie (11c) von der Seite der Mittelachse (L5) nähert, und beide Endabschnitte (11d, 11e) der Querschnittslinie (11c) an gleichen Positionen in einer Richtung angeordnet sind, in der sich die Mittelachse (L5) erstreckt.

## Revendications

1. Tube de fixation de globe oculaire (1, 8) qui est temporairement fixé à une membrane extérieure d'un globe oculaire en chirurgie ophtalmique en vue d'une communication entre un intérieur et un extérieur du globe oculaire, le tube de fixation de globe oculaire (1, 8) comportant :
une partie tubulaire (2, 9) ayant une forme tubulaire, la partie tubulaire (2, 9) étant configurée pour être dans un état de perforation de la membrane extérieure lorsque le tube de fixation de globe oculaire (1, 8) est fixé à la membrane extérieure ; et
une partie de grand diamètre (3, 10) disposée sur un côté d'extrémité proximale de la partie tubulaire (2, 9), la partie de grand diamètre (3, 10) ayant un diamètre extérieur plus grand qu'un diamètre extérieur de la partie tubulaire (2, 9), la partie de grand diamètre (3, 10) étant configurée pour être exposée extérieurement à partir de la membrane extérieure lorsque le tube de fixation de globe oculaire (1, 8) est fixé à la membrane extérieure, dans lequel
une partie d'extrémité (31, 11), dans une direction parallèle à un axe central (L1, L5) de la partie tubulaire (2, 9), de la partie de grand diamètre (3, 10) sur le côté de la partie tubulaire (2, 9) est formée de manière à présenter une différence de hauteur dans la direction parallèle entre une première extrémité (31a, 1 la) et une seconde extrémité (31b, 11b) dans une direction perpendiculaire à l'axe central (L1, L5) dans une vue de côté,
une totalité de la partie d'extrémité (31, 11) est formée comme un plan incliné par rapport à l'axe central (L1, L5), et
une partie d'extrémité (32, 12) de la partie de grand diamètre (3, 10), sur un côté opposé à la partie d'extrémité (31, 11) sur le côté de la partie tubulaire (2, 9) dans la direction parallèle est formée comme un plan perpendiculaire à l'axe central (L1, L5), dans lequel la partie de grand diamètre (3, 10) inclut une valve (35) pour empêcher du liquide de s'écouler à l'extérieur depuis une ouverture (34) formée dans la partie d'extrémité (32, 12) sur le côté opposé,
dans lequel la valve (35) est un élément ayant une fente formée dans un matériau élastique de manière à fermer un canal (33) dans la partie de grand diamètre (3, 10).

2. Tube de fixation de globe oculaire (1) selon la revendication 1, dans lequel le plan incliné (31) est un plan plat.

3. Tube de fixation de globe oculaire (8) selon la revendication 1, dans lequel
le plan incliné (11) est une surface courbe évidée,
sur une première coupe transversale, lorsqu'elle est vue dans une même direction que dans la vue de côté, obtenue en coupant le tube de fixation de globe oculaire (8) au niveau d'un plan incluant l'axe central (L5) dans celui-ci, la surface courbe évidée (11) est formée de manière à être déplacée graduellement vers une extrémité avant de la partie tubulaire (9) à mesure que la surface courbe évidée (11) s'approche d'un côté de la seconde extrémité (11b) depuis le côté de la première extrémité (11a) dans la direction perpendiculaire à l'axe central (L5), et à rendre une ligne courbe oblique par rapport à l'axe central (L5), et
sur une seconde coupe transversale, obtenue en coupant le tube de fixation de globe oculaire (8) au niveau d'un plan perpendiculaire à la première section transversale, incluant l'axe central (L5) dans celui-ci, une ligne de coupe transversale (11c) rendue par la surface courbe évidée (11) est graduellement déplacée vers la partie d'extrémité (12) sur le côté opposé de manière à former une ligne courbe à mesure que la ligne de coupe transversale (11c) s'approche de l'axe central (L5) à partir de l'une des parties d'extrémité (11d, 11e) de la ligne de coupe transversale (11c), et graduellement déplacée vers l'extrémité avant de la partie tubulaire (9) de manière à former une ligne courbe à mesure que la ligne de coupe transversale (11c) s'approche d'une autre des parties d'extrémité (11d, 11e) de la ligne de coupe transversale (11c) à partir du côté de l'axe central (L5), et les deux parties extrémité (11d, 11e) de la ligne de coupe transversale (11c) sont disposées aux mêmes positions dans une direction dans laquelle l'axe central (L5) s'étend.
